(19)

Europäisches
Patentamt
European
Patent Office
Office européen
des brevets

(11)  **EP 4 201 320 A1**

(12)  **EUROPEAN PATENT APPLICATION**

(43) Date of publication:
**28.06.2023  Bulletin 2023/26**

(51) International Patent Classification (IPC):
**A61B 5/06** (2006.01)      **A61B 5/276** (2021.01)
**A61B 5/304** (2021.01)

(21) Application number: **21216810.8**

(22) Date of filing: **22.12.2021**

(52) Cooperative Patent Classification (CPC):
**A61B 5/061; A61B 5/276; A61B 5/304;**
A61B 2560/0276

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **LEIJSSEN, Jacobus Josephus
Eindhoven (NL)**
• **VAN UDEN, Martijn Gerarda Lambertus Justinus
Eindhoven (NL)**

(74) Representative: **Philips Intellectual Property &
Standards
High Tech Campus 52
5656 AG Eindhoven (NL)**

(54)  **INTERVENTIONAL DEVICES**

(57)  A mechanism for determining to which of a plurality of device electrodes a respective plurality of connection pins is communicatively coupled. For each connection pin, a measure of an electrical parameter is obtained. The electrical parameter is a parameter that changes responsive to a change in distance between the device electrode (connected to the connection pin) and a calibration electrode. The measures are processed to identify the device electrode to which the connection pin is communicatively coupled.

200

FIG. 2

EP 4 201 320 A1

**Description**

FIELD OF THE INVENTION

The present invention relates to the field of interventional devices, and in particular, to interventional devices carrying three or more device electrodes.

BACKGROUND OF THE INVENTION

There is an increasing interest in the (computer) modelling of anatomical structures of a subject, to provide a visual representation of the anatomical structure. This aids analysis of the anatomical structure, e.g. during the course of a medical procedure or diagnosis.

One area of particular interest is in mapping of an anatomical structure (such as the heart), in which an interventional device is inserted into the anatomical cavity of the anatomical structure and one or more navigation and/or mapping systems are used to localize the device, which can then be used to generate an anatomical model of the anatomical cavity or structure.

One example of a suitable mapping system is the EPD Solutions navigation system provided by Philips®, named the KODEX-EPD® system, which exploits electric field sensing for 3D localization, allowing radiation-free navigation in an anatomical cavity, e.g. inside the heart.

Some mapping techniques, such as those employed by the KODEX-EPD system, are based on electrical fields between three pairs of electrode-patches that represent the x-, y-, and z-dimension. Voltages at electrodes carried by an interventional device (device electrodes) are sampled, and a real-time voltage-to-position function is formed. This function is continuously updated based on the measurements coming in. Once sufficiently updated, the voltage-to-position function enables the system to reliably display the spatial movement of the device.

One downside to existing mapping systems is that there is a need to provide reliable electrical connections between device electrodes and an external processing system. As mapping systems are designed to be generic, e.g. for different types of interventional device, this usually necessitates the use of a pin box. An operator will traditionally need to correctly connect each device electrode to a correction connection pin, in order for the mapping system to know from which device electrode samples/measurements are being obtained.

There is an ongoing desire to reduce the burden of an operator, to prevent erroneous corrections and therefore avoid any potential misinterpretation.

SUMMARY OF THE INVENTION

The invention is defined by the claims.

According to examples in accordance with an aspect of the invention, there is provided a processing system for predicting, for each of three or more connection pins, with which of a corresponding number of device electrodes the connection pin is communicatively coupled, each device electrode being an electrode carried by an interventional device.

The processing system comprises: an input interface configured to receive: for each connection pin, a measure of an electrical parameter that changes responsive to a distance between a calibration electrode and the device electrode communicatively coupled to the connection pin, wherein the calibration electrode is either an external electrode not carried by the interventional device or another one of the device electrodes not connected to the connection pin; a processing unit communicatively coupled to the input interface and configured to process at least all obtained measures of the electrical parameter to predict, for each connection pin, the device electrode to which the connection pin is communicatively coupled; and optionally, an output interface configured to provide output data identifying, for each connection pin, the predicted device electrode to which the connection pin is communicatively coupled.

The proposed processing system thereby provides a mechanism for automatically determining to which device electrode a connection pin is communicatively coupled, i.e. the mapping between connection pins and device electrodes. This significantly reduces a burden on an operator of the interventional device (or a system comprising the same) to correctly connect the device electrodes of the interventional device to the connection pin(s). This approach also advantageously avoids any errors in connection, which would propagate into any later processes that make use of the device electrodes.

The proposed mechanism is particularly advantageous as it can be performed highly efficiently using existing circuitry, components and capabilities of interventional devices and interventional device systems in which such devices are used. Thus, additional expense and/or risk for an operator is further reduced.

In some examples, the input interface is further configured to receive positional information identifying the positional relationship between the device electrodes; and the processing unit is configured to process the positional information and all obtained measures of the electrical parameter to predict, for each connection pin, the device electrode to which the connection pin is communicatively coupled.

In this way, known information about how the device electrodes are positionally or spatially related to one another can be used to determine the mapping between connection pins and device electrodes. For instance, an order of the device electrodes on the interventional device (e.g. a tip to base order) can be used in the determination of the mapping.

The electrical parameter may be an electrical impedance between the device electrode and the calibration electrode.

As another example, the electrical parameter may be an amplitude of either: a difference between the voltage induced at the device electrode as a result of an electric field induced by the calibration electrode and a voltage at a reference electrode or the calibration electrode; and a difference between the voltage induced at the device electrode as a result of an electric field induced by an electric field generator, and the voltage induced at the calibration electrode as a result of the electric field induced by the electric field generator.

As yet another example, the electrical parameter is a phase of an electrical signal or a phase difference between electrical signals.

For instance, the electrical parameter may be a phase difference between an electrical signal produced at the device electrode, as a result of an electric field induced by the calibration electrode, and an electrical signal provided to the calibration electrode to produce the electric field.

Alternatively, the electrical parameter may be a phase difference between an electrical signal produced at the device electrode, as a result of an electric field induced by an electric field generator, and an electrical signal produced at the calibration electrode, as a result of the electric field induced by the electric field generator.

In some examples, the input interface is configured to receive: identifying information that identifies the device electrode connected to a subset of the three or more connection pins, the subset comprising not all of the three or more connection pins; for each connection pin, a set of measurements, each measurement being a measure of an electrical parameter that changes responsive to a change in distance the device electrode to which the connection pin is connected and another device electrode of the interventional device, wherein, for each connection pin, the set of measurements contains a measurement for each other device electrode of the interventional device; and the processing unit is configured to process the identifying information all received sets of measurements to predict, for each connection pin, the device electrode to which the connection pin is communicatively coupled.

The identifying information may be useful for resolving an unknown degree of freedom. In particular, the identifying information may be used to resolve or identify which side of the interventional device (e.g. closer to the tip or closer to the base) an electrode for a particular connection pin is located.

In some examples, the processing unit is configured to: identify, from all received sets of measurements, a first measurement, the first measurement being the measurement that represents the greatest distance between any device electrode and any other device electrode; and predict that the connection pins communicatively coupled to the two device electrodes associated with the first measurement are communicatively coupled to the device electrodes located the furthest distance away from one another.

Optionally, the processing unit is configured to: define a first set of device electrodes, comprising the device electrodes associated with the identified measurement; define a second set of device electrodes, comprising the device electrodes not contained in the first set of device electrodes; perform a first iterative process comprising: identifying, from all received sets of measurements, a second measurement, the second measurement being the measurement that represents the smallest distance between any device electrode in the second set and any of the device electrodes in the first set; predicting that the connection pin communicatively coupled to the device electrode of the second set, associated with the second measurement, is communicatively coupled to the device electrode positioned most proximately to the device electrode of the first set associated with the second measurement; replacing the device electrode in the first set associated with the second measurement with the device electrode of the second set associated with the second measurement; and removing the device electrode of the second set associated with the second measurement from the second set.

The iterative process may further comprise: identifying, from all received sets of measurements, a third measurement, the third measurement being the measurement that represents the smallest distance between any device electrode in the second set not associated with the second measurement and any of the device electrodes in the first set not associated with the second measurement; predicting that the connection pin communicatively coupled to the device electrode of the second set, associated with the third measurement, is communicatively coupled to the device electrode positioned most proximately to the device electrode of the first set associated with the third measurement; replacing the device electrode in the first set associated with the third measurement with the device electrode of the second set associated with the third measurement; and removing the device electrode of the second set associated with the third measurement from the second set.

Preferably, the device electrodes are linearly positioned on the interventional device.

There is also proposed a kit for predicting, for each of three or more connection pins, with which of a corresponding number of device electrodes the connection pin is communicatively coupled, the kit comprising: the processing system as previously described; and a chamber sized to house at least the portion of the interventional device carrying the three or more different device electrodes

The chamber may comprise a fluid having a relative permittivity greater than 60.

There is also proposed a computer-implemented method for predicting, for each of three or more connection pins, with which of a corresponding number of device electrodes the connection pin is communicatively coupled, each device electrode being an electrode carried by an interventional device.

The computer-implemented method comprises: receiving at an input interface, for each connection pin, a measure of an electrical parameter that changes responsive to a distance between a calibration electrode and the device electrode communicatively coupled to the connection pin, wherein the calibration electrode is either an external electrode not carried by the interventional device or another one of the device electrodes not connected to the connection pin; processing, using a processing unit communicatively coupled to the input interface, at least all obtained measures of the electrical parameter to predict, for each connection pin, the device electrode to which the connection pin is communicatively coupled.

The computer-implemented method may also comprise providing, at an output interface, output data identifying, for each connection pin, the predicted device electrode to which the connection pin is communicatively coupled.

There is also proposed a computer program product comprising computer program code means which, when executed on a computing device having a processing system, cause the processing system to perform all of the steps of any herein described method.

Any herein described method may be adapted to perform the steps executed by any herein described processing system and/or processing unit, and vice versa.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1    illustrates a system;
Figure 2    is a flowchart illustrating a method;
Figure 3    illustrates an approach employed by embodiments;
Figure 4    illustrates another approach employed by embodiments;
Figure 5    illustrates yet another approach employed by embodiments;
Figure 6    illustrates a kit;
Figure 7    illustrates a processing system; and
Figure 8    illustrates a processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for determining to which of a plurality of device electrodes a respective plurality of connection pins is communicatively coupled. For each connection pin, a measure of an electrical parameter is obtained. The electrical parameter is a parameter that changes responsive to a change in distance between the device electrode (connected to the connection pin) and a calibration electrode. The measures are processed to identify the device electrode to which the connection pin is communicatively coupled.

Embodiments are based on the realization that the position of a device electrode with respect to the interventional device (i.e. the identity of the device electrode) affects an electrical parameter that changes with respect to distance between the device electrode and a calibration electrode. This facilitates identification of the device electrode from/by processing measures of this electrical parameter.

Disclosed approaches can be employed to automatically determine or predict to which respective device electrode each of a plurality of connection pins is connected. This avoids a need for an operator or operation team of an interventional device (system) to perform specific electrode to connection pin coupling, thereby reducing a burden on the operator or operation team and reducing error in processes that make use of monitoring electrical responses of device electrodes.

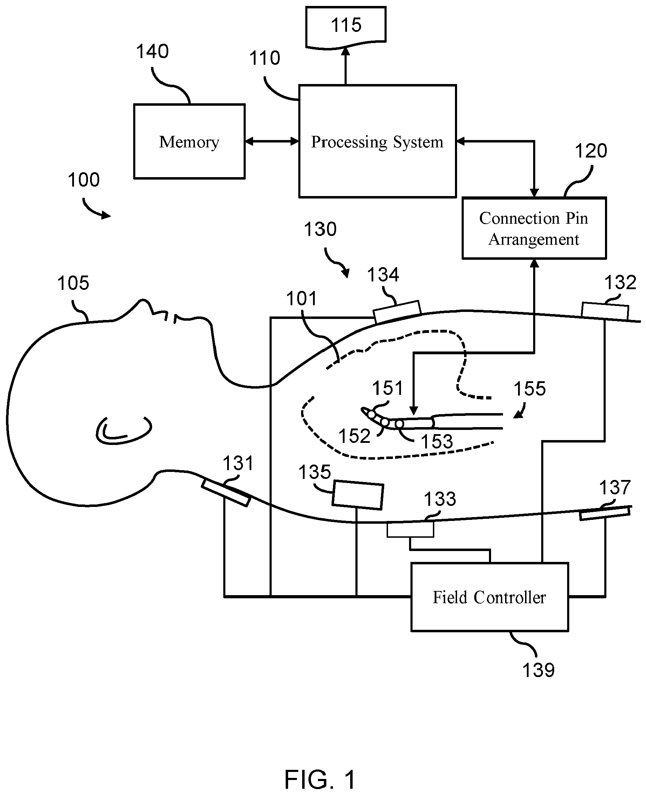

Figure 1 conceptually illustrates a system 100 in which embodiments can be employed. The system 100 comprises a processing system 110, according to embodiments of the invention, and an interventional device 155.

The interventional device 155 is configured to enter and move within an anatomical cavity 101 of a subject 105. The interventional device 155 carries a set of three or more device electrodes, i.e. a plurality of three or more device electrodes 151, 152, 153. The device electrodes are positioned along a line, i.e. linearly. Each device electrode is configured to connect to the processing system 110, via a connection pin arrangement 120, so that the electrical signals at the device electrode can be controlled and/or analyzed/sampled by the processing system 110.

Each device electrode is configured to connect (e.g. via a respective wire or lead) to a respective connection pin of the connection pin arrangement 120, which acts as an inputting interface for the processing system 110. The connection pin arrangement 120 may therefore be formed of a plurality of connection pins to which leads/wires (connected to device electrodes of the interventional device) can be coupled. The connection pins may, for instance, be female connectors with the leads, connected to the device electrodes, having male connectors (or vice versa).

Signals detected/sensed by the device electrodes 151, 152, 153 can be used to track the location of each device electrode and therefore the interventional device. In particular, the electrical response of each device electrode of the interventional device to crossing electric fields induced within the subject can be monitored and mapped to a location in a predefined co-ordinate system (sometimes called an R-space) using an appropriate mapping function. This mapping function is used at least partly because induced electrical fields' distribution is inherently inhomogeneous due to the different dielectric properties and absorption rates (related to conductivity) of the interrogated tissues.

The crossing electric fields may be generated by a field generating system 130, formed of a plurality of external electrodes 131, 132, 133, 134, 135, 137 positionable around the subject 105. The external electrodes may, for instance, comprise electrode patches or patch electrodes. The electric signal provided to each external electrode is controllable by a field controller 139, to thereby control the electric fields between the external electrodes.

Approaches for generating a mapping function are well established in the art. Example processes are disclosed by the European Patent Applications EP 0775466 A2, EP 3568068 A1 and EP 3607879 A1. Typically, generating a mapping function makes use of known inter-electrode distances to calibrate for the inhomogeneity in the crossing electric fields.

However, in additional to simply sensing signals at a device electrode, it may also be desired to control and/or define signals provided by a device electrode. This could allow, for instance, for ablation to be applied by a device electrode or for electrical parameters of anatomical matter surrounding the device electrode to be determined (e.g. by monitoring a current flow between different device electrodes). The processing system 110 may accordingly be configured to control signals provided to the device electrodes via the connection pin arrangement 120.

In current system set-ups, the interventional device 155 is often a single-use component, to reduce cross-contamination and meet disinfection and sterilization requirements. However, it would be inefficient to provide single use processing systems 110 for analyzing and/or controlling signals at the device electrodes.

It is therefore usually necessary for an operator of the system 100 to manually connect (the wires/leads attached to) device electrodes 151, 152, 153 to respective connection pins of the connection pin arrangement 120. It will be appreciated that this is a difficult task to perform accurately, especially for large numbers of device electrodes. Future development of interventional devices may see devices carrying 50 or more device electrodes, e.g. 100 or more device electrodes.

For existing systems, improper connection or misalignment between the device electrodes and the connection pin arrangement would result in the processing system attributing incorrect measurements to incorrectly connected device electrodes. This would have a significant impact on the further processing of such information.

For instance, generation of a mapping function would be performed inaccurately (as such generation usually relies upon known inter-electrode distances which would not align for incorrectly connected device electrodes).

As another example, attempted ablation using an incorrectly connected device electrode would result in an incorrect device electrode performing ablation, which could cause unintended damage to the subject.

Other negative consequences of incorrectly connected device electrodes would be apparent to the skilled person.

There is therefore a significant burden on the operator of the system 100 to correctly position the device electrodes, especially if there are an extremely large number of device electrodes.

Proposed embodiments provide approaches for automatically predicting, for each connection pin, with which device electrode that connection pin is communicatively coupled. In other words, automatic determination of the mapping between device electrodes and connection pins is facilitated. This would significantly reduce a burden on an operator of the system and reduce or account for any potential error in the connecting of the device electrodes 151, 152, 153 to the connection pin arrangement 120.

Disclosed concepts make use of measures, for each connection pin, of an electrical parameter that changes responsive to distance between a device electrode and a calibration electrode. This information facilitates identification of the order or relative spatial position of each device electrode within the set of device electrodes.

Embodiments are based on the realization that such information can be used to determine the order or spatial relationship of the device electrodes associated with each pin collector. In other words, this information can be used to identify the mapping between device electrodes and connection pins.

Conceptually, the spatial position of a device electrode 151, 152, 153 connected to a connection pin (with respect to other device electrodes) may be initially unknown to a processing system 110 receiving input from the connection pin arrangement 120. The measures of the electrical parameter allow the processing system to establish the spatial relationship between the devices connected to the connection pins.

For the following examples, it is assumed that the device electrodes are positioned linearly along the interventional device. This facilitates ease of identifying to which device electrode each connection pin is connected by processing appropriate electrical parameter(s).

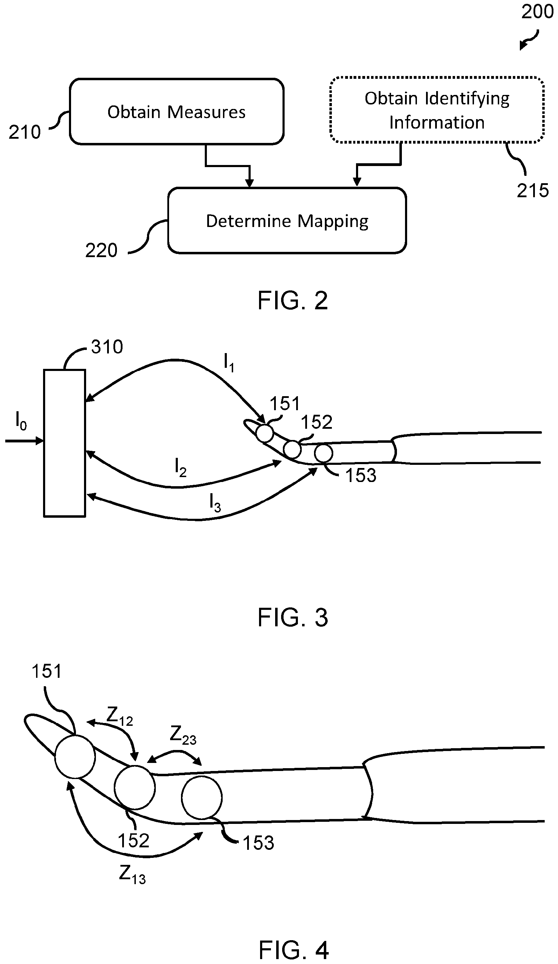

Figure 2 illustrates a computer-implemented method 200 according to embodiments of the invention. The method may be implemented by a processing system, as will be later described.

The method 200 comprises a step 210 of receiving information. Step 210 may be performed by an input interface of the processing system.

Step 210 comprises receiving, for each connection pin, a measure of an electrical parameter that changes responsive to a distance between a calibration electrode and the device electrode communicatively coupled to the connection pin. The calibration electrode is either an external electrode not carried by the interventional device or another one of the device electrodes not connected to the connection pin.

The obtained measures of the electrical parameter facilitate identification of at least how to correctly order the connection pins to match the true order of the device electrodes on the interventional device.

For instance, the obtained measures can be used to determine which connection pins are associated with device electrodes that neighbor one another. If it is known to which device electrode one of the connection pins is connected, it is possible to construct or build up a mapping between each device electrode and each connection pin.

As another example, the obtained measures can be used to determine a measure that indicates a distance from a tip of the interventional device, facilitating ordering of the connection pins by distance from the tip (which, in turn, can be used to order the connection pins in a defined order for the device electrodes).

The method 200 also comprises a step 220 of processing all obtained measures of the electrical parameter to predict, for each connection pin, the device electrode to which the connection pin is communicatively coupled. Thus, in step 220, the information obtained in step 210 is processed to establish a mapping between the device electrodes and the connection pins.

Step 220 may comprise determining or identifying, for each connection pin, which one or more other connection pins are connected to a device electrode that spatially neighbors the device electrode connected to said connection pin. In this way, the connection pins can be conceptually ordered by the order of device electrodes to which they are connected.

Hereafter described are various and non-exhaustive examples of electrical parameters that change responsive to a distance between the calibration electrode and the device electrode communicatively coupled to the connection pin.

Generally, these electrical parameters fall into one of four types. A first type is an electrical parameter that is determined/derived by monitoring, for a device electrode, a signal induced at the device electrode by a signal provided to an external electrode. A second type is an electrical parameter that is determined/derived by monitoring, for a device electrode, a signal at device electrode and a signal at an external electrode that have both been induced by an electric field generated by a field generator. A third type is an electrical parameter that is derived from monitoring a signal induced at a second device electrode by a signal provided to a first device electrode. A fourth type is an electrical parameter that is derived from monitoring a signal at a first device electrode and a signal at a second device electrode that have been induced by a signal provided to an external electrode.

The first and second types represent electrical parameters that change with distance between an external electrode and a device electrode. The third and fourth types represent electrical parameters that change with distance between device electrodes.

All of these example types provide examples of electrical parameters, for a device electrode, that change with distance of the device electrode from a calibration electrode, in which the calibration electrode is either another of the device electrodes or an external electrode.

Figure 3 illustrates the principle of one approach for determining to which device electrode each connection pin is connected. Figure 3 is used to illustrate examples of the first and second type of electrical parameter described above.

In this approach, the electrical parameter is an electrical parameter that is responsive to a distance from an external electrode 310 to each device electrode 151, 152, 153.

The external electrode 310 may, for instance, be one of the electrodes that generate crossing electric fields, as illustrated in Figure 1. In particular, the external electrode 310 is preferably one that is at a known position relative to the position and movement of the interventional device during the determination of the mapping between connection pins and device electrodes.

In a first example, the electrical parameter (obtained for each connection pin) is a phase delay of an electrical signal induced in the device electrode 151, 152, 153 (connected to the connection pin) by an external electrode 310.

A current $I_0$ is provided to the external electrode 310, so that a current $I_1$, $I_2$, $I_3$ flows from the external electrode to each device electrode 151, 152, 153 (and subsequently to a respective connection pin). A phase difference (or "phase shift") between the current at the external electrode 310 and the current at a device electrode will change depending upon the distance between the external electrode and device electrode. The greater the distance, the greater the phase difference.

By way of explanation, the relationship between the velocity of an electric signal in a material with the permittivity of the material can be approximated as follows:

$$V \cong \frac{c}{\sqrt{E_r}} \qquad\qquad (1)$$

where c is the speed of light into air, $E_r$ is the permittivity of the material and V the propagation speed of the electrical signal through the material. It will be appreciated that the actual formula is more complex than the simplification of equation (1). Thus, the propagation of an electrical current $I_1$, $I_2$, $I_3$ through the material is increasingly delayed with increasing permittivity $E_r$ of the material.

The phase difference/shift obtained for each connection pin can be used to predict to which device electrode that connection pin is communicatively coupled.

Increasing phase difference indicates increasing distance from the external electrode 330. Thus, if a connection pin is associated with the smallest phase shift, then it can be determined that this connection pin is connected to the device electrode closest to the external electrode (e.g. is an electrode closest to a tip of the interventional device). Similarly, if a connection pin is associated with the largest phase shift, then it can be determined that this connection pin is connected to the device electrode further to the external electrode (e.g. is an electrode closest to a base of the interventional device). Phase shifts lying between these values can be used to appropriately map the associated connection pin to device electrodes.

A synchronic detector could be used to determine the value of the phase shift of an electrical signal between emission by the external electrode 310 and receipt by the device electrode 151, 152, 153. The value of the phase shift changes responsive to distance between the external electrode and the device electrode.

To improve ease of identifying the phase shift of the electrical signal, the interventional device may be immersed in a material with a relatively high (e.g. >60) permittivity. Advantageously, this material may be a sterilizing liquid, e.g. to (further) sterilize the interventional device before insertion into a subject. However, this is not essential.

As another example, the material may be material of the subject (e.g. anatomical matter). The relative permittivity of human tissue (or blood) is in the region of 70 and 80. This makes the flow of electrical current roughly 8-9 times slower than the speed of an electrical current a traditional conductive wire. This approach increases an ease of measuring electrical speed of signals into the electrodes, e.g. compared to through air.

In a second example, the electrical parameter (obtained for each connection pin) is a magnitude of the current induced in the device electrode by the signal provided to the external electrode.

As previously explained, a current $I_0$ can be provided to the external electrode 310, so that a respective current $I_1$, $I_2$, $I_3$ flows from the external electrode to each device electrode 151, 152, 153 (and subsequently to a respective connection pin). The magnitude of each current $I_1$, $I_2$, $I_3$ that flows from the external electrode to each respective device electrode 151, 152, 153 is dependent upon the impedance between the external electrode and each device electrode, and therefore the distance from the external electrode to each device electrode.

In a third example, the electrical parameter (obtained for each connection pin) is a magnitude of a voltage induced in the device electrode by the signal provided to the external electrode.

Providing a current $I_0$ to an external electrode will generate an electric field. This generated electric field will induce a voltage in each device electrode. The magnitude of the induced voltage will be responsive to the distance between the electric field and the device electrode. In this way, the magnitude of the induced voltage will represent a distance between the external electrode and the device electrode.

The voltage may be a voltage difference between the device electrode and a ground/reference electrode or a voltage between the device electrode and the external electrode. Both are inversely related to the distance between the external electrode and the device electrode.

In a fourth example, the electrical parameter is a voltage difference between a signal induced at the external electrode 310 and a signal induced at a device electrode by an electric field generated by an electric field generator. The magnitude of this voltage difference will increase responsive to increased distance between the external electrode and the device electrode.

If the interventional device is appropriately aligned and positioned with respect to the external electrode, the ordering of the connection pins can be easily performed based on the determined electrical parameter. For instance, if the electrical parameter is a phase difference, then increasing phase difference indicates increasing distance from the external electrode.

Generally, for an interventional device that is linear, device electrodes are identified by their distance or order from the tip of the interventional device. The distance of each device electrode from the external electrode can effectively represent or indicate its position in the order, thereby allowing the processing system to establish to which device electrode each connection pin is connected.

For instance, the connection pin that is connected to the device electrode identified as being closest to the external electrode could be identified as being connected to the electrode closest to the tip of the interventional device (as it can be assumed that the tip will be most proximate to the external electrode). Similarly, increasing distance from the external electrode may indicate that the connection pin is connected to increasingly less distal device electrodes.

Thus, the measures of the electrical parameter facilitate ordering of the connection pins by the associated distance from the external electrode, this order representing the corresponding order of the device electrodes.

Figure 4 illustrates another the principle of another approach for determining to which device electrode each connection pin is connected. Figure 4 is used to illustrate examples of the third and fourth types of electrical parameter described above.

In this example, the electrical parameter is an electrical parameter that is responsive to a distance between two device electrodes 151, 152, 153.

Suitable examples of such an electrical parameter include an impedance $Z_{12}$, $Z_{13}$, $Z_{23}$, capacitance and/or resistance between two device electrodes 151, 152, 153. Other examples include a voltage difference (for voltages induced by an electric field) or a current difference (for current induced in the electrodes from an external current source).

An impedance between a pair of device electrodes can be determined by supplying (at a particular frequency for that pair) a positive voltage to one of the pair and a negative voltage to the other of the pair. Monitoring the voltage at each of the pair of device electrodes and the current therebetween facilitates determination of the impedance between the two device electrodes, using Ohm's law.

It is possible to determine the impedances at a same time if each pair of device electrodes is associated with a different frequency, as this allows specific current flow between the device electrodes to be distinguished and monitored separately from the others, e.g. using frequency de-multiplexing.

In another example, differences in the phase difference between the different device electrodes could be used. The phase difference may be calculated as previously explained with reference to Figure 3.

As another example, differences in the voltage response (to an external electric field) of each device electrode could be used. The voltage response may be calculated as previously explained with reference to Figure 3.

In yet another example, the electrical parameter responsive to a distance between two device electrodes (first and second device electrodes) may be a voltage difference between a first signal supplied to the first device electrode and a second signal induced in the second device electrode by the first signal. The magnitude of this voltage difference will increase for increased distance between the device electrodes.

In yet another example, the electrical parameter responsive to a distance between two device electrodes (first and second device electrodes) may be phase difference between a first signal suppled to the first device electrode and a second signal induced in the second device electrode by the first signal. The magnitude of the phase difference will increase with increased distance between the device electrodes.

Referring back to Figure 2, for this approach, step 210 may be modified such that step comprises obtaining a measure for this electrical parameter for each pair of device electrodes. Thus, if there are N device electrodes, then N! / (2.(N-2)!) measures would be obtained by step 210.

These measures can be first processed to identify the connection pins communicatively coupled to the two device electrodes at the two ends of the interventional device. This can be performed by identifying, from all received sets of measurements, a first measurement, the first measurement being the measurement that represents the greatest distance between any device electrode and any other device electrode. The two device electrodes associated with the first measurement are the device electrodes located the furthest distance away from one another. Thus, the connection pins associated with the most distant electrodes can be identified.

The (remaining) measures can then be iteratively processed to identify which connection pin(s) is/are associated with the next most proximate device electrodes to those for whom the mapping between connection pin and device electrode has been established. In this way, the connection pins can be arranged in the spatial order that the device electrodes to which they are connected are carried by the interventional device. The ordering is performed from the most distant device electrodes inwardly.

For instance, this iterative process may be performed by first defining two sets of device electrodes. Initially, a first set comprises the device electrodes associated with the first measurement. Initially, a second set comprises all other device electrodes. In the iterative process, the measurements are processed to identify the nearest device electrode of the second set to any of the device electrodes of the first set or the nearest two device electrodes of the second set to any two different of the device electrodes of the first set. The nearest device electrode(s) are then removed from the second set and added to the first set. This iterative process is then repeated.

The approach described with reference to Figure 4 identifies the appropriate end-to-end order for the connection pins, but is not able to directly identify which device electrode is at the tip of the interventional device, and which is at the base. In other words, it is unknown whether the order is tip-to-base or base-to-tip.

Identifying information could be used to establish a correct mapping between at least one of the device electrodes (e.g. a device electrode at the tip of the interventional device) and the connection pin to which it is connected. The identifying information identifies the device electrode connected to a subset of the three or more connection pins, the subset comprising not all of the three or more connection pins.

For instance, the identifying information may identify a mapping between a single device electrode (e.g. the tip device electrode or the furthest from the tip device electrode) and a corresponding connection pin.

If there is an even number of device electrodes, the mapping between any single one of the device electrodes can be used to correct determine the tip-to-base order of the device electrodes. If there is an even number of device electrodes, the mapping between any single one of the device electrodes excluding the central electrode can be used to correct extrapolate the tip-to-base order of the device electrodes.

Referring back to Figure 2, the method 200 may further comprise a step 215 of obtaining the identifying information.

The identifying information may have been originally defined, for instance, as a result of a user action or input. For instance, an operator of the system 100 may be guided to always insert or connect at least one of the device electrodes to a predetermined connection pin (e.g. by way of pattern, color or other visual indicator). In this way, the identifying information may be knowledge that a certain connection pin will always correspond to a predetermined one of the device electrodes. For instance, the predetermined one of the device electrodes may be the device electrode nearest the tip of the interventional device.

Whilst this approach relies upon some user action, the burden on the operator is significantly reduced (e.g. as only a select number, e.g. 1, of connection pins need to be correctly coupled to the corresponding device electrode).

As another example, identifying information may be determined by monitoring the electrical response of the device electrodes as the interventional device is inserted (i.e. tip-first) into a material/liquid (e.g. blood or disinfecting fluid). The electrical response of an electrode (e.g. to its own emitted electrical signal) will change upon contact with a different material. If the interventional device is inserted tip-first, the device electrode that responds to this change first (as monitored by the connection pins) will be the tip electrode, facilitating generation of the identifying information.

Other suitable examples will be apparent to the skilled person.

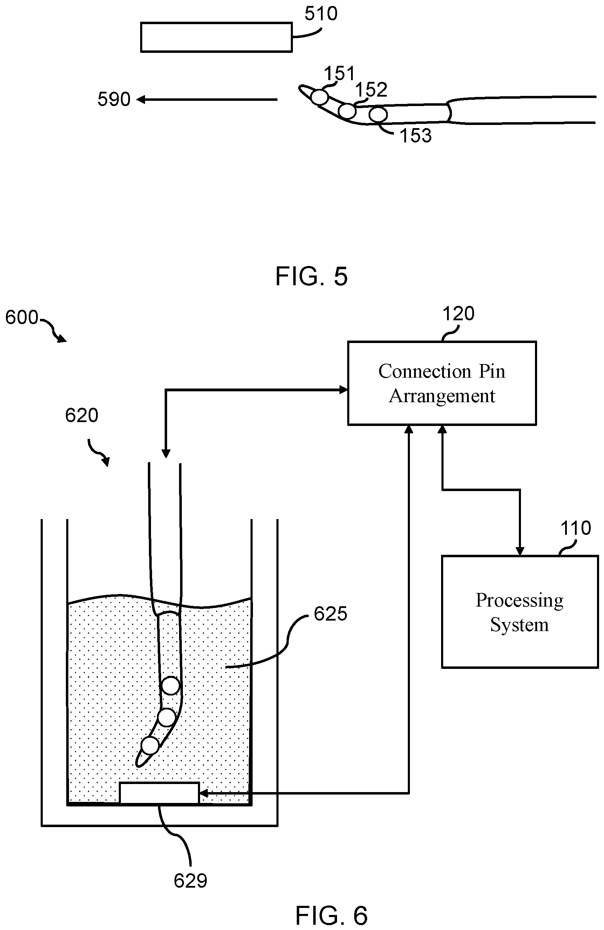

Figure 5 illustrates the principle of yet another approach for determining to which device electrode each connection pin is connected.

In this example, the calibration electrode is an external electrode 510. For each connection pin, an electrical parameter is monitored over time, wherein the electrical parameter that changes responsive to a distance between a calibration electrode and the device electrode communicatively coupled to the connection pin.

Suitable examples for the electrical parameter include phase difference of a signal provided at the external electrode 510 and the signal received at the device electrode 151, 152, 153 to which the connection pin is connected. Another example is an impedance, capacitance or resistance between the external electrode 510 and the device electrode to which the connection pin is connected. Another example is a voltage difference between the external electrode and the device electrode.

The interventional device 155 is moved in a direction 590 alongside the external electrode. The movement is performed so that each device electrode, in turn, becomes the most proximate device electrode to the external device electrode over the course of the movement.

The measurements of the parameter are monitored, to track the order in which the device electrode for each connection pin becomes the most proximate to the external electrode. This effectively facilitates identification of the spatial arrangement of the device electrodes with respect to the connection pins, and thereby determination of the mapping.

For instance, the first device electrode to come close to the external electrode (as monitored via the measurements of the electrical parameter) can be determined to be the electrode at the tip of the interventional device, and the connection pin may be marked or labelled accordingly. The next device electrode can to come close to the external electrode (as monitored via the measurements of the electrical parameter) can be determined to be the electrode at the tip of the interventional device, and the connection pin may be marked or labelled accordingly.

This process can be repeated until the mapping of all connection pins to device electrodes is complete.

Any above-described method can be performed using measures of the electrical parameter that are gathered when the interventional device is not placed inside the subject. For instances, the measures of the electrical parameter could be obtained when the interventional device is placed in a dedicated calibration module or chamber.

However, in other examples, an above-described method is performed whilst the interventional device is within the subject, e.g. surrounded by blood.

Figure 6 illustrates a kit 600 according to embodiments of the invention. The kit 600 comprises a processing system 110 and a chamber 620.

The processing system 110 is itself an embodiment of the invention, and is configured to perform any previously described method for predicting, for each of three or more connection pins, with which of a corresponding number of device electrodes the connection pin is communicatively coupled.

The chamber 620 is sized to house at least the portion of the interventional device 155 carrying the three or more device electrodes. The chamber 620 may comprise a fluid 625 having relative permittivity greater than 60, e.g. greater than 70. The amount of fluid and/or the size of the chamber may be configured such that the fluid surrounds the inserted portion of the interventional device, such that the fluid lies completely surrounds the three or more device electrodes.

The chamber 620 may further comprise an electrode 629, which can be used as a calibration electrode for use in previously described embodiments (e.g. as a source of electric current for deriving phase difference measurements). The electrode 629 may be controlled and/or coupled to the processing system 110, e.g. via the connection pin arrangement 120.

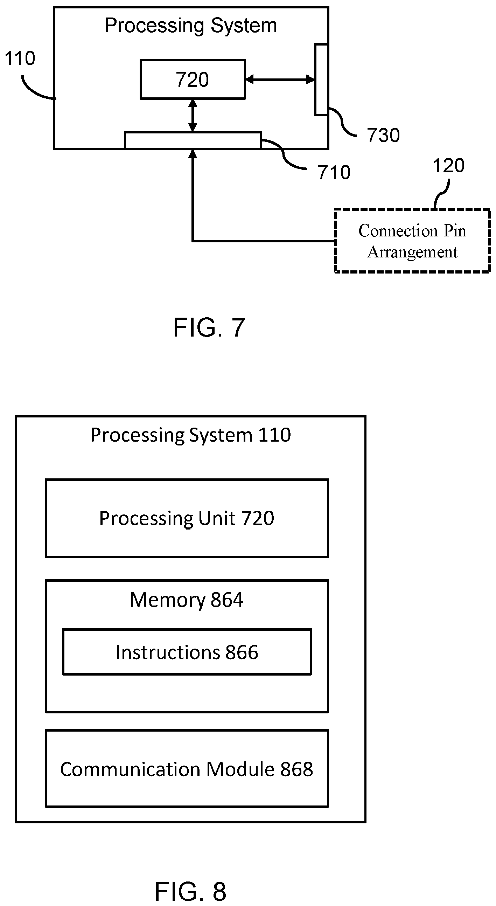

Figure 7 illustrates the processing system 110 according to embodiments. The processing system is configured for predicting, for each of three or more connection pins, with which of a corresponding number of device electrodes the connection pin is communicatively coupled, each device electrode being an electrode carried by an interventional device.

The processing system 110 comprises an input interface 710 configured to obtain identifying information that identifies the device electrode connected to a subset of the three or more connection pins, the subset comprising not all of the three or more connection pins.

The input interface 710 is also configured to obtain, for each connection pin, a measure of an electrical parameter that changes responsive to a distance between a calibration electrode and the device electrode communicatively coupled to the connection pin, wherein the calibration electrode is either an external electrode not carried by the interventional device or another one of the device electrodes not connected to the connection pin;

The measure(s) of the electrical parameter may be obtained from the connection pin arrangement 120 formed of the three or more connection pins. Alternatively, the information may be obtained from a memory. Of course, a combination of these possibilities could be used.

The processing system 110 also comprises a processing unit 720 communicatively coupled to the input interface.

The processing unit is configured to process the identifying information and all obtained measures of the electrical parameter to predict, for each connection pin, the device electrode to which the connection pin is communicatively coupled. Thus, the processing unit determines a mapping between the connection pins of the connection pin arrangement and the device electrodes of the interventional device.

The processing system 110 may be appropriately adapted to perform any herein described method, as would be readily apparent to the skilled person. For instance, the input interface 710 may be appropriately adapted to perform any embodiment of step 210, and the processing unit 720 may be appropriately adapted to perform any embodiment of step 220.

The processing system may further comprise an output interface 730 configured to provide output data identifying, for each connection pin, the predicted device electrode to which the connection pin is communicatively coupled. Thus, the output interface 730 may provide the mapping determined by the processing unit 720.

The output data may be provided to a further processing system, e.g. which uses the determined mapping (between connection pins and device electrodes) to perform further processes.

For instance, this information may be used in the generation of a mapping function that maps an electrical response of an electrode to a position within a predefined co-ordinate system (as previously described). This is because the generation of such a mapping function may rely upon known inter-electrode distances.

As another example, this information may be used to control an ablation procedure performed using the interventional device, e.g. to make sure that ablation is performed by desired electrodes.

Figure 8 is a schematic diagram of a processing system 110, according to embodiments of the present disclosure. The processing system 110 is configured to carry out a method according to an embodiment, such as the method 200 described with reference to Figure 2.

As shown, the processing system 110 may include a (data) processing unit 720, a memory 864, and a communication module 868. These elements may be in direct or indirect communication with each other, for example via one or more buses.

The processing unit 720 may include a central processing unit (CPU), a digital signal processor (DSP), an ASIC, a controller, an FPGA, another hardware device, a firmware device, or any combination thereof configured to perform the operations described herein. The processing unit 720 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 864 may include a cache memory (e.g., a cache memory of the processing unit 720), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 864 includes a non-transitory computer-readable medium. The non-transitory computer-readable medium may store instructions. For example, the memory 864, or non-transitory computer-readable medium may have program code recorded thereon, the program code including instructions for causing the processing system 110, or one or more components of the processing system 110, particularly the processing unit 720, to perform the operations described herein. For example, the processing system 110 can execute operations of the method 800.

Instructions 866 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 864, with the code recorded thereon, may be referred to as a computer program product.

The communication module 868 can include any electronic circuitry and/or logic circuitry to facilitate direct or indirect communication of data between the processing system 110, the penetration device and/or the user interface (or other further device). In that regard, the communication module 868 can be an input/output (I/O) device. In some instances, the communication module 868 facilitates direct or indirect communication between various elements of the processing system 110.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored there on the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

**Claims**

1. A processing system (110) for predicting, for each of three or more connection pins, with which of a corresponding number of device electrodes (151, 152, 153) the connection pin is communicatively coupled, each device electrode being an electrode carried by an interventional device (150), the processing system comprising:
an input interface (710) configured to receive (210):

for each connection pin, a measure of an electrical parameter that changes responsive to a distance between a calibration electrode and the device electrode communicatively coupled to the connection pin, wherein the calibration electrode is either an external electrode not carried by the interventional device or another one of the device electrodes not connected to the connection pin;
a processing unit (720) communicatively coupled to the input interface and configured to process (220) at least all obtained measures of the electrical parameter to predict, for each connection pin, the device electrode to which the connection pin is communicatively coupled; and
optionally, an output interface (730) configured to provide output data identifying, for each connection pin, the predicted device electrode to which the connection pin is communicatively coupled.

2. The processing system of claim 1, wherein

the input interface is further configured to receive positional information identifying the positional relationship between the device electrodes; and
the processing unit is configured to process the positional information and all obtained measures of the electrical parameter to predict, for each connection pin, the device electrode to which the connection pin is communicatively coupled.

3. The processing system of claim 1 or 2, wherein the electrical parameter is an electrical impedance between the device electrode and the calibration electrode.

4. The processing system of claim 1 or 2, wherein the electrical parameter is an amplitude of either:

a difference between the voltage induced at the device electrode as a result of an electric field induced by the calibration electrode and a voltage at a reference electrode or the calibration electrode; and
a difference between the voltage induced at the device electrode as a result of an electric field induced by an electric field generator, and the voltage induced at the calibration electrode as a result of the electric field induced by the electric field generator.

5. The processing system of claims 1 or 2, wherein the electrical parameter is a phase of an electrical signal or a phase difference between electrical signals.

6. The processing system of claim 5, wherein the electrical parameter is a phase difference between an electrical signal produced at the device electrode, as a result of an electric field induced by the calibration electrode, and an electrical signal provided to the calibration electrode to produce the electric field.

7. The processing system of claim 5, wherein the electrical parameter is a phase difference between an electrical signal produced at the device electrode, as a result of an electric field induced by an electric field generator, and an electrical signal produced at the calibration electrode, as a result of the electric field induced by the electric field generator.

8. The processing system of any of claims 1 to 7, wherein:
the input interface is configured to receive:

identifying information that identifies the device electrode connected to a subset of the three or more connection pins, the subset comprising not all of the three or more connection pins;
for each connection pin, a set of measurements, each measurement being a measure of an electrical parameter that changes responsive to a change in distance the device electrode to which the connection pin is connected and another device electrode of the interventional device,
wherein, for each connection pin, the set of measurements contains a measurement for each other device electrode of the interventional device; and

the processing unit is configured to process the identifying information all received sets of measurements to predict, for each connection pin, the device electrode to which the connection pin is communicatively coupled.

9. The processing system of claim 8, wherein the processing unit is configured to:

identify, from all received sets of measurements, a first measurement, the first measurement being the measurement that represents the greatest distance between any device electrode and any other device electrode; and predict that the connection pins communicatively coupled to the two device electrodes associated with the first measurement are communicatively coupled to the device electrodes located the furthest distance away from one another.

10. The processing system of claim 9, wherein the processing unit is configured to:

define a first set of device electrodes, comprising the device electrodes associated with the identified measurement;
define a second set of device electrodes, comprising the device electrodes not contained in the first set of device electrodes;
perform a first iterative process comprising:

identifying, from all received sets of measurements, a second measurement, the second measurement being the measurement that represents the smallest distance between any device electrode in the second set and any of the device electrodes in the first set;
predicting that the connection pin communicatively coupled to the device electrode of the second set, associated with the second measurement, is communicatively coupled to the device electrode positioned most proximately to the device electrode of the first set associated with the second measurement;
replacing the device electrode in the first set associated with the second measurement with the device electrode of the second set associated with the second measurement; and
removing the device electrode of the second set associated with the second measurement from the second set.

11. The processing system of claim 10, wherein the iterative process further comprises:

identifying, from all received sets of measurements, a third measurement, the third measurement being the measurement that represents the smallest distance between any device electrode in the second set not associated with the second measurement and any of the device electrodes in the first set not associated with the second measurement;
predicting that the connection pin communicatively coupled to the device electrode of the second set, associated with the third measurement, is communicatively coupled to the device electrode positioned most proximately to the device electrode of the first set associated with the third measurement;
replacing the device electrode in the first set associated with the third measurement with the device electrode of the second set associated with the third measurement; and
removing the device electrode of the second set associated with the third measurement from the second set.

12. The processing system of any of claims 1 to 11, wherein the device electrodes are linearly positioned on the interventional device.

13. A kit for predicting, for each of three or more connection pins, with which of a corresponding number of device electrodes the connection pin is communicatively coupled, the kit comprising:

the processing system of any of claims 1 to 12; and
a chamber sized to house at least the portion of the interventional device carrying the three or more different device electrodes, and comprising a fluid having a relative permittivity greater than 60.

14. A computer-implemented method (200) for predicting, for each of three or more connection pins, with which of a corresponding number of device electrodes (151, 152, 153) the connection pin is communicatively coupled, each device electrode being an electrode carried by an interventional device (150), the computer-implemented method comprising:

receiving (210) at an input interface, for each connection pin, a measure of an electrical parameter that changes responsive to a distance between a calibration electrode and the device electrode communicatively coupled to the connection pin, wherein the calibration electrode is either an external electrode not carried by the interventional device or another one of the device electrodes not connected to the connection pin;

processing (220), using a processing unit communicatively coupled to the input interface, at least all obtained measures of the electrical parameter to predict, for each connection pin, the device electrode to which the connection pin is communicatively coupled; and

optionally providing, at an output interface, output data identifying, for each connection pin, the predicted device electrode to which the connection pin is communicatively coupled.

**15.** A computer program product comprising computer program code means which, when executed on a computing device having a processing system, cause the processing system to perform all of the steps of the method according to claim 14.

**FIG. 1**

200

FIG. 2

FIG. 3

FIG. 4

FIG. 5

FIG. 6

Processing System

110

720

730

710

120

Connection Pin
Arrangement

## FIG. 7

Processing System 110

Processing Unit 720

Memory 864

Instructions 866

Communication Module 868

## FIG. 8

Europäisches
Patentamt

European
Patent Office

Office européen
des brevets

# EUROPEAN SEARCH REPORT

Application Number

EP 21 21 6810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/152420 A1 (ST JUDE MEDICAL CARDIOLOGY DIV INC [US]) 8 August 2019 (2019-08-08) * paragraph [0072] - paragraph [0092] * ----- | 1-15 | INV. A61B5/06 A61B5/276 A61B5/304 |
| A | WO 2017/192775 A1 (ACUTUS MEDICAL INC [US]) 9 November 2017 (2017-11-09) * paragraph [0138] - paragraph [0145] * ----- | 1-15 | |

TECHNICAL FIELDS
SEARCHED        (IPC)

A61B

The present search report has been drawn up for all claims

| Place of search | Date of completion of the search | Examiner |
|---|---|---|
| The Hague | 15 June 2022 | Van Dop, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 6810

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2022

| Patent document cited in search report | | Publication date | Patent family member(s) | | Publication date |
|---|---|---|---|---|---|
| WO 2019152420 | A1 | 08-08-2019 | US 2021076968 A1 | | 18-03-2021 |
| | | | WO 2019152420 A1 | | 08-08-2019 |
| WO 2017192775 | A1 | 09-11-2017 | AU 2017260453 A1 | | 22-11-2018 |
| | | | CA 3022806 A1 | | 09-11-2017 |
| | | | CN 109715055 A | | 03-05-2019 |
| | | | CN 114129172 A | | 04-03-2022 |
| | | | EP 3451917 A1 | | 13-03-2019 |
| | | | EP 3973908 A1 | | 30-03-2022 |
| | | | JP 6937321 B2 | | 22-09-2021 |
| | | | JP 2019514578 A | | 06-06-2019 |
| | | | JP 2021183199 A | | 02-12-2021 |
| | | | US 2019200886 A1 | | 04-07-2019 |
| | | | WO 2017192775 A1 | | 09-11-2017 |

EPO FORM P0459

For more details about this annex : see Official Journal of the European Patent Office, No. 12/82

**EP 4 201 320 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0775466 A2
- EP 3568068 A1
- EP 3607879 A1